# EUROPÄISCHE PATENTANMELDUNG

(11) **EP 1 764 083 A1**
(43) Veröffentlichungstag der Anmeldung: **21.03.2007**
(21) Anmeldenummer: 06120668.6
(22) Anmeldetag: 14.09.2006
(51) Int. Cl.: A61K 8/72, A61Q 19/04, A61Q 17/00

(54) **Sonnenschutzmittel mit Ligninsulfonaten**

(30) Priorität: 16.09.2005 DE 102005045144
(71) Anmelder: Beiersdorf AG, 20253 Hamburg (DE)
(72) Erfinder: Lerg, Heike, 22303, Hamburg (DE); Mundt, Claudia, 28207, Bremen (DE); Schäfer, Jessica, 22299, Hamburg (DE); Sugár, Martin, 20257, Hamburg (DE); Windisch, Björna, 22067, Hamburg (DE)

(57) **Zusammenfassung**

Kosmetische Zubereitung enthaltend ein oder mehrere UV-Lichtschutzfilter sowie Ligninsulfonsäure und/oder Ligninsulfonate.

## Beschreibung

Die vorliegende Erfindung betrifft eine kosmetische Zubereitung enthaltend ein oder mehrere UV-Lichtschutzfilter sowie Ligninsulfonsäure und/oder Ligninsulfonate.

Der Trend weg von der vornehmen Blässe hin zur "gesunden, sportlich braunen Haut" ist seit Jahren ungebrochen. Um eine gebräunte Hautfarbe zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Für den umfassenden Schutz der menschlichen Haut vor der UV-Strahlung ist es entscheidend, dass das Sonnenschutzmittel lückenlos und in gleichmäßiger Menge auf die Haut aufgetragen wird. In der Regel ist es dem Verbraucher jedoch nicht möglich, die Auftragungsmenge zu kontrollieren. Als Folge dieses Mangels werden mangelhaft geschützte Hautpartien einer übermäßigen Strahlenbelastung ausgesetzt; es kommt zu Hautrötungen und Sonnenbrand. Es war daher die Aufgabe der vorliegenden Erfindung, eine vor der UV-Strahlung schützende kosmetische Zubereitung zu entwickeln, die sich für den Anwender leicht und kontrollierbar gleichmäßig auf die Haut auftragen lässt.

Nach dem Stande der Technik versucht man eine gleichmäßige Auftragung einer kosmetischen Zubereitung dadurch zu erleichtern, dass man der Zubereitung Farbstoffe, beispielsweise Eisenoxide, zusetzt. Derartige Formulierungen haben jedoch den Nachteil, dass sie der Haut ein unnatürliches, maskenhaftes Aussehen verleihen. Es ist dann gut zu erkennen, das ein farbgebendes Produkt angewendet wurde, was insbesondere Männer von der Anwendung derartiger Produkte abhält. Auch besitzen derartige farbgebene Pigmente ein Größe von - 10 - 50 µm und liegen daher auf der Haut auf. Dies führt dann regelmäßig zu einem Abfärben der Partikel auf die auf der Haut getragene Kleidung. Es war daher die Aufgabe der vorliegenden Erfindung, eine kosmetische Zubereitung zu entwickeln, deren Verteilung auf der Haut durch Zusatz von Farbstoffen sichtbar wird, bei der andererseits der Farbstoff nicht auf die Kleidung abfärbt und der Haut kein maskenhaftes Aussehen verleiht.

Hauptanwender von kosmetischen Sonnenschutzprodukten sind die sogenannten "Sonnenanbeter", d.h. Menschen, die das mehr oder weniger intensive Bad in der Sonne genießen. Ein Hauptaspekt beim Sonnenbad ist dabei die Bräunung der Haut. Sonnenanbeter sind dabei dem Zwiespalt ausgesetzt, einerseits die Haut bräunen zu wollen, was den ungehinderten Kontakt der Haut mit dem UV-Licht voraussetzt, andererseits die Haut vor den negativen Einflüssen des UV-Lichts (Sonnenbrand, vorzeitige Hautalterung, Hautkrebs) schützen zu wollen, was einen Einsatz an UV-Lichtschutzfiltern unumgänglich macht. Als Alternative zum Sonnenbad stehen dem Verbraucher zur Hautbräunung sogenannte "Selbstbräuner" (in der Regel Dihydroxyaceton, DHA) zur Verfügung. Bei der Anwendung von DHA vergeht jedoch relativ viel Zeit, bis eine Hautbräunung sichtbar wird. Auch weicht der Braunton, welcher durch DHA auf der Haut erzeugt wird, in der Regel stark vom natürlichen Braunton sonnengebräunter Haut ab. Die Haut wird eher gelb als braun. Es war daher die Aufgabe der vorliegenden Erfindung eine kosmetische Zubereitung zu entwickeln, welche die Haut schnell mit einem natürlichen Braunton verleiht.

Kosmetische Sonnenschutzmittel sind hoch komplexe Substanzmischungen, deren häufig mangelnde Homogenität und Stabilität bei der Lagerung und Anwendung (UV-Stabilität) den Kosmetiker immer wieder vor Probleme stellt.

So ist aus der Familie der UV-A-Filtersubstanzen (im folgenden auch UV-A-Filter genannt) die Verbindungsklasse der Dibenzoylmethanderivate, insbesondere 4-(tert.-Butyl)-4'-methoxydiben-zoylmethan als besonders leistungsstarke UV-Filter bekannt. Leider ist von dieser Verbindungsklasse ebenfalls bekannt, dass die Filter selbst relativ empfindlich gegenüber der UV-Strahlung sind und die Tendenz zeigen, sich unter der Einwirkung des UV-Lichtes mehr oder weniger schnell zu zersetzen. Die Photostabilität von Dibenzoylmethanderivaten ist also begrenzt und die UV-Lichtschutzfilterleistung nimmt bei der Anwendung der Filter auf der Haut im Verlaufe der Zeit ab. Es war daher die Aufgabe der vorliegenden Erfindung eine Zubereitung zu entwickeln, in welcher 4-(tert.-Butyl)-4'-methoxydibenzoylmethan stabilisiert ist und die einen lang anhaltenden UV-A-Schutz mit hoher UV-A-Balance gewährleistet.

Nicht zuletzt war es die Aufgabe der vorliegenden Erfindung, eine Zubereitung zu entwickeln, welche alle der beschriebenen Aufgaben gleichzeitig löst.

Überraschend gelöst werden die Aufgaben durch eine kosmetische Zubereitung enthaltend
a) ein oder mehrere UV-Lichtschutzfilter sowie
b) Ligninsulfonsäure und/oder Ligninsulfonate.

Die erfindungsgemäßen Zubereitungen weisen gegenüber Zubereitungen ohne Ligninsulfonate einen höheren Lichtschutzfaktor (LSF) und eine höhere UV-A Balance (nach DIN 67502) auf.

Durch die erfindungsgemäßen Zubereitungen wird das gleichmäßige Auftragen von Sonnenschutzmitteln auf der Haut erleichtert und die Haut in attraktiver Weise vorgebräunt ohne dass bei dieser Prozedur die Gefahr besteht, dass durch Abrieb von Zubereitung die nach der Anwendung mit der Haut in Kontakt geratene Kleidung verschmutzt wird.

Durch die Anwendung der erfindungsgemäßen Zubereitung wird der PPD-Wert (persistent pigment darkening Wert) deutlich erhöht und die Haut zusätzlich vor Austrocknung geschützt.

In den erfindungsgemäßen Zubereitungen wird die Photostabilität des UV-Lichtschutzfilters 4-(tert.-Butyl)-4'-methoxydibenzoylmethan (CAS-Nr. 70356-09-1) erhöht.

Zwar beschreibt die WO 2002/013608 Lignin als wasserlösliches UV-Schutzmaterial für ein bioaktives Pestizid, doch konnte diese Schrift den Weg zur vorliegenden Erfindung ebenso wenig weisen wie die WO 95/33378, welche den Schutz biologischer Pestizide mit Ligninen vor Hitze und UV-Strahlung beschreibt.

Es ist erfindungsgemäß vorteilhaft, wenn die erfindungsgemäße Zubereitung UV-Lichtschutzfilter in einer Konzentration von 0,1 bis 30 Gewichts-% und bevorzugt in einer Konzentration von 1 bis 20 Gewichts-%, jeweils bezogen auf das Gesamtgewicht der Zubereitung, enthält.

Ferner ist es erfindungsgemäß vorteilhaft, wenn die erfindungsgemäße Zubereitung Ligninsulfonsäure und/oder Ligninsulfonate in einer Gesamtkonzentration von 0,01 bis 20 Gewichts-% und bevorzugt in einer Konzentration von 1 bis 15 Gewichts-%, jeweils bezogen auf das Gesamtgewicht der Zubereitung, enthält.

Es ist erfindungsgemäß bevorzugt, wenn in der erfindungsgemäßen Zubereitung Ligninsulfonate (z.B. als Litium-, Natrium-, Ammonium-, Kalium-, Calcium-, Magnesium-, Barium-, Strontium-Salze) eingesetzt werden.

Erfindungsgemäß besonders bevorzugt ist es, wenn als Ligninsulfonat Natriumligninsulfonat (CAS 8061-51-6) eingesetzt wird.

Außerdem ist es erfindungsgemäß bevorzugt, wenn das erfindungsgemäße Ligninsulfonat ein durchschnittliches Molekulargewicht von 500 bis zu 10000 Dalton (insbesondere von etwa 5000 Dalton) aufweist.

Als erfindungsgemäß vorteilhaftes Ligninsulfonat kann beispielsweise das Produkt Vanispere CB der Firma Borregaard Industries eingesetzt werden.

Die erfindungsgemäßen UV-Lichtschutzfilter können erfindungsgemäß vorteilhaft gewählt werden aus der Gruppe der UV-Lichtschutzfilter 2,4-Bis-{[4-(2-ethyl-hexyloxy)-2-hydroxy]-phenyl}-6-(4-methoxyphenyl)-1,3,5-triazin; Dioctylbutylamidotriazon; 2,4-bis-[5-1 (dimethylpropyl)benzoxazol-2-yl-(4-phenyl)-imino]-6-(2-ethylhexyl)-imino-1,3,5-triazin; 4,4',4"-(1,3,5-Triazin-2,4,6-triyltriimino)-tris-benzoësäure-tris(2-ethylhexylester); 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; Ethylhexylsalicylat; Terephthalidendicamphersulfonsäure; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalon-säuredi(2-ethylhexyl)- ester; Dioctylbutylamidotriazon; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dimethicodiethylbenzalmalonat; 4-Methoxyzimtsäure(2-ethylhexyl)ester; 4-(tert.-Butyl)-4'-methoxydibenzoylmethan; Titandioxid; Zinkoxid.

Erfindungsgemäß bevorzugte Ausführungsformen der vorliegenden Erfindung enthalten ein oder mehrere UV-Lichtschutzfilter gewählt aus der Gruppe der Triazine, Titandioxid.

Auch ist es bevorzugt im Sinne der vorliegenden Erfindung, wenn die erfindungsgemäße Zubereitung den UV-Lichtschutzfilter 4-(tert.-Butyl)-4'-methoxydibenzoylmethan (CAS-Nr. 70356-09-1) enthält.

Erfindungsgemäß vorteilhafte Ausführungsformen der vorliegenden Erfindung sind dadurch gekennzeichnet, dass die Zubereitung Selbstbräuner (insbesondere Dihydroxyaceton, DHA) enthält.

Erfindungsgemäß vorteilhaft beträgt das Gewichtsverhältnis der UV-Lichtschutzfilter zur Gesamtmenge an Ligninsulfonsäure und/oder Ligninsulfonate erfindungsgemäß von 2:1 bis 1:2.

Erfindungsgemäße kosmetische Zubereitungen können in verschiedenen Formen vorliegen. So können sie z.B. eine Lösung, eine wasserfreie Zubereitung, eine Dispersion, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/OM/), ein Gel, einen festen Stift, eine Salbe, einen Schaum oder auch ein Aerosol darstellen.

Dabei ist es erfindungsgemäß bevorzugt, wenn die kosmetische Zubereitung in Form einer Emulsion und besonders bevorzugt, wenn die Zubereitung in Form einer O/W-Emulsion vorliegt. Bei der Herstellung der Emulsionen oder Dispersionen können alle bekannten Emulgatoren und Emulgatorsysteme problemlos verwendet werden. Vorteilhaft ist bei Verwendung eines sehr lipophilen Emulgators geringe Mengen an hydrophilem Emulgator zuzusetzen (z.B. 0,5% eine PEG Stearates).

Die erfindungsgemäße Zubereitung kann erfindungsgemäß vorteilhaft als Salbe, Creme oder Lotion (ggf. sprühbar) vorliegen.

Die erfindungsgemäße Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Emulsion, erfindungsgemäß.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner (z.B. Dihydroxyaceton), Repellentien sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die Ölphase der erfindungsgemäßen Zubereitung kann alle herkömmlichen Bestandteile von in der Kosmetik eingesetzten Öl-, Fett- und Wachskomponenten enthalten.

Die erfindungsgemäße Zubereitung kann erfindungsgemäß vorteilhaft kosmetische Wirk- und Pflegestoffe enthalten, beispielsweise die nach der Kosmetikverordnung zugelassenen Konservierungsmittel oder Konservierungshelfer. Derartige Wirkstoffe können erfindungsgemäß vorteilhaft in Konzentrationen von 0,01 bis 30 Gewichts-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Licochalkon und/oder alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Die erfindungsgemäße Zubereitung kann beliebige Mischungen von Duft- und Parfümstoffen in den unterschiedlichsten Konzentrationen enthalten.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

### Beispiele

### O/W-Emulsionen: Hoher Schutz (high protection), SPF 20

| **INCI** | **1** | **2** | **3** | **4** | **5** | **6** | **7** | **8** | **9** |
|---|---|---|---|---|---|---|---|---|---|
| Alcohol Denat. | 3 | 4 | 2 | 0 | 3 | 5 | 5 | 5 | 5 |
| Acrylates/C10-30 Alkyl Acrylate Crosspolymer | 0,1 | 0,0 5 | 0,2 | 0,0 5 | 0 | 0 | 0 | 0 | 0 |
| Polyglyceryl-2 Dipolyhydroxystearate | 0,2 5 | 0,5 | 0,6 | 0,5 | 0 | 0 | 0 | 0 | 0 |
| Glyceryl Stearate Citrate | 1 | 2 | 3 | 2,5 | 0 | 0 | 0 | 0 | 0 |
| Glyceryl Stearate SE | 0 | 0 | 0 | 0 | 2 | 1 | 1 | 1 | 1 |
| Cetearyl Alcohol + PEG-40 Castor Oil + Sodium Cetearyl Sulfate | 0 | 0 | 0 | 0 | 5 | 2,5 | 2,5 | 2,5 | 2,5 |
| VP/Hexadecene Copolymer | 0,5 | 0,5 | 0,4 | 0,5 | 0,2 | 0,5 | 0,5 | 0,5 | 0,5 |
| Trisodium EDTA | 1 | 0,5 | 1 | 1 | 1 | 1 | 1 | 1 | 1 |
| Preservatives | q.s. | q.s. | q.s. | q.s. | q.s. | q.s. | q.s. | q.s. | q.s. |
| Terephthalidene Dicamphor Sulfonic Acid | | | | | | | 5 | 10 | 10 |
| Drometrizole Trisiloxane | | | | | | | 5 | 5 | 5 |
| Bis-Ethylhexyloxyphenol Methoxyphenyl Triazine | 0,5 | 1,4 | 1 | 2 | 0,5 | 1,4 | | | |
| Disodium Phenyl Dibenzimidazole Tetrasulfonate | 0 | 1 | 0 | 1,5 | 0 | 0 | 0 | 0 | 0 |
| Ethylhexyl Triazone | 1 | 1,6 | 3,5 | 4 | 0 | 0 | 0 | 0 | 0 |
| Butyl Methoxydibenzoylmethane | 4,5 | 3,5 | 3,5 | 3 | 4,5 | 4,5 | 4,5 | 4,8 | 4,8 |
| Ethylhexyl Methoxycinnamate | 8 | 8 | 0 | 0 | 6 | 5 | | | |
| 2,4-bis-[5-1(dimethylpropyl)benzoxazol-2-yl-(4-phenyl)-imino]-6-(2- ethylhexyl)-imino-1,3,5-triazin | 0 | 1,5 | 0 | 0 | 0 | 0 | 0 | 5 | 3 |
| Aminobenzophenon | 1 | 0 | 0 | 0 | 0 | 0 | 0 | 4 | 8 |
| Octocrylene | 0 | 0 | 0 | 0 | 2 | 3 | 10 | 10 | 10 |
| Phenylbenzimidazole Sulfonic Acid | 0 | 0 | 0 | 0 | 2 | 2 | | | |
| Ethylhexylglycerin | 0,5 | 0,5 | 0,5 | 0,5 | 0,5 | 0,5 | 0,5 | 0,5 | 0,5 |
| Glycerin | 5 | 7,5 | 7,5 | 6 | 2 | 6 | 8 | 8 | 8 |
| Hydrogenated Coco-Glycerides | 1 | 0 | 1 | 1 | 1 | 1 | 1 | 1 | 1 |
| Butylene Glycol Dicaprylate/Dicaprate | 3 | 2 | 9 | 8 | 4 | 2 | 2 | 2 | 2 |
| Dicaprylyl Ether | 2 | 3 | 1 | 4 | 1 | 2 | 2 | 2 | 2 |
| C12-15 Alkyl Benzoate | 5 | 8 | 7,5 | 6 | 5 | 4 | 4 | 4 | 4 |
| Dibutyl Adipate | 0 | 0 | 3 | 0 | 2 | 1 | 1 | 1 | 1 |
| Dihydroxyaceton (DHA) | 5 | | | 2 | | | 1 | | |
| Parfum | q.s. | q.s. | q.s. | q.s. | q.s. | q.s. | q.s. | q.s. | q.s. |
| Titanium Dioxide + Trimethoxycaprylylsilane | 3 | 3 | 3 | 3 | 3 | 3 | 5 | 5 | 5 |
| Cyclomethicone | 0 | 2 | 1 | 2 | 3 | 0 | 0 | 0 | 0 |
| Xanthan Gum | 0,2 | 0,1 | 0,4 | 0,2 | 0,4 | 0,25 | 0,25 | 0,25 | 0,25 |
| Stearyl Alcohol | 1 | 1,5 | 2 | 1,2 | 1 | 1,5 | 1,5 | 1,5 | 1,5 |
| Aqua | ad 100 | ad 100 | ad 100 | ad 100 | ad 100 | ad 100 | ad 100 | ad 100 | ad 100 |
| Natrium Ligninsulfonate | 3 | 5 | 2 | 1,5 | 8 | 2,5 | 0,5 | 2,75 | 3,5 |
| Creatinine | 0,05 | 0,1 | 0,03 | 0,05 | 0,06 | 0,05 | 0,05 | 0,05 | 0,05 |
| Dihydroxyacteon | | 3 | 5 | | | | | | |
| Tocopheryl Acetate | 0,5 | 0,2 | 0,5 | 0,5 | 0,5 | 0,5 | 0,5 | 0,5 | 0,5 |
| Creatine | 0,5 | 1 | 0,3 | 0,5 | 0,6 | 0,5 | 0,5 | 0,5 | 0,5 |
| Ascorbic Acid | | | | | | | 1,0 | 4,0 | 0,5 |
| Natrium-Ascorbyl Phosphate | | | | | 0,8 | 0,2 | | | |

### O/W Emulsionen

| | **10** | **11** | **12** | **13** | **14** | **15** | **16** |
|---|---|---|---|---|---|---|---|
| PEG-40 Castor Öl, Natrium Cetearyl Sulfat, Cetearyl Alkohol | 2,50 | 3,50 | 2,50 | 2,25 | 2,00 | 3,00 | 2,50 |
| Glycerinmonostearat (SE) | 1,00 | 1,50 | 1,00 | 1,25 | 1,50 | 1,00 | 1,00 |
| Cetyl Alkohol | 1,50 | 1,00 | 1,50 | 2,00 | 1,00 | 1,00 | 1,50 |
| Uvinul® A Plus | | | | | 4,00 | 2,00 | |
| Butylmethoxydibenzoylmethan | 4,90 | 4,50 | 2,00 | 3,50 | 2,50 | 3,50 | 4,50 |
| 4-Methylbenzylidencampher | | | | | | | |
| Bis-Ethylhexyloxyphenol Methoxyphenyl Triazin | 1,50 | 3,00 | 2,00 | 2,00 | | 1,00 | 3,00 |
| Dinatrium Phenyl Dibenzimidazol Tetrasulfonat | | | | | | | |
| Phenylbenzmidazol Sulfonsäure | | 2,00 | 2,00 | | | 2,50 | 2,00 |
| Ethylhexyl Triazon | | 1,50 | | 3,00 | | | |
| Diethylhexyl Butamido Triazon | 1,00 | | 0,50 | 1,00 | 1,50 | | |
| Ethylhexyl Methoxycinnamat | 4,50 | | 3,00 | 4,50 | | | 9,50 |
| Octocrylen | 3,00 | | | 1,00 | | | |
| Ethylhexylsalicylat | | | | 4,50 | 3,50 | 2,00 | |
| Polysilicon 15 | | | | | | 2,00 | |
| Titandioxid T 805 | | | | | | | 1,00 |
| C₁₂₋₁₅ Alkyl Benzoat | 5,50 | 5,50 | 3,50 | 5,50 | 4,50 | 5,50 | 5,50 |
| Octyldodecanol | 5,50 | 5,50 | 7,50 | 5,50 | 4,50 | 5,50 | 5,50 |
| Risocast DA-H | 2,00 | 0,50 | 1,00 | 1,50 | 3,00 | 6,00 | 1,75 |
| Dicaprylylether | | | | | | 1,00 | |
| Paraffinöl | | 2,00 | | | | | |
| Butylenglycol Dicaprylat/ Dicaprat | 1,00 | | | | | | |
| Cetearyl Isononanoat | | | | | 2,00 | | |
| Dimethicon | | | 2,00 | | | | |
| Cyclomethicon | | | 5,00 | 3,00 | | | |
| Dimethicon / Vinyl Dimethicon Crosspolymer | | | | | 0,30 | | |
| PVP Hexadecen Copolymer | | | | | | 0,50 | |
| Glycerin | 7,50 | 1,00 | 2,00 | 1,00 | 1,00 | 1,00 | 1,00 |
| Ligninsulfonate | 1 | 2,5 | 1,5 | 4 | 3 | 3,5 | 1 |
| Methylpropandiol | | | 2,00 | | 1,00 | | |
| Butylenglycol | | | | | | 2,00 | |
| Vitamin E Acetat | 2,00 | 0,10 | 0,50 | 0,10 | | 0,75 | |
| Retinol | | | | | | | |
| Natrium Stärke Octenylsuccinat | 1,50 | 1,50 | 1,50 | | 1,50 | 1,00 | 1,50 |
| Maisstärke | 2,00 | 3,00 | | 1,00 | 3,00 | 4,00 | 3,00 |
| Dihydroxyaceton | | | 2,00 | | | | |
| Methylparaben | 0,10 | 0,10 | 0,20 | | 0,10 | | 0,10 |
| Phenoxyethanol | 0,60 | 0,50 | 0,60 | 0,60 | 0,60 | | 0,60 |
| EDTA | 0,20 | 0,20 | 0,20 | 0,20 | 0,20 | 0,20 | 0,20 |
| Ethanol | 8,00 | 8,00 | 8,00 | 8,00 | 8,00 | 8,00 | 8,00 |
| Insekt Repellent 3535 | | | | 0,50 | | | |
| Parfüm | 0,40 | 0,40 | 0,40 | 0,40 | 0,40 | 0,40 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 5,0-7,5 | 5,0-7,5 | 5,0-7,5 | 5,0-7,5 | 5,0-7,5 | 5,0-7,5 | 5,0-7,5 |

### W/O-Emulsionen: Hoher Schutz (high protection), SPF 20

| **INCI** | **1** | **2** | **3** | **4** | **5** | **6** |
|---|---|---|---|---|---|---|
| Alcohol Denat. | 1 | 2 | 1 | 2 | 1 | 2 |
| Polyglyceryl-2 Dipolyhydroxystearate | 4 | 5,5 | 4 | 5,5 | 4 | 5,5 |
| Trisodium EDTA | 1 | 1 | 1 | 1 | 1 | 1 |
| Preservatives | q.s. | q.s. | q.s. | q.s. | q.s. | q.s. |
| Paraffinum Liquidum | 0 | 1,8 | 0 | 1,8 | 0 | 1,8 |
| Ethylhexyl Triazone | | | 1 | | | |
| Diethylhexyl Butamido Triazone | 0,5 | 0 | | | | |
| Bis-Ethylhexyloxyphenol Methoxyphenyl Triazine | 1 | 1 | | | | |
| 2,4-bis-[5-1 (dimethylpropyl)benzoxazol-2-yl-(4-phenyl)-imino]-6-(2- ethylhexyl)-imino-1,3,5-triazin | | 0,5 | | | 9 | 8 |
| Aminobenzophenon | | 0,5 | 0,8 | | 6,4 | 5,5 |
| Octocrylene | 2,5 | 6 | 10 | 10 | 10 | 10 |
| Terephthalidene Dicamphor Sulfonic Acid | | | 8 | | 8 | |
| Drometrizole Trisiloxane | | | 12 | | 12 | |
| Disodium Phenyl Dibenzimidazole Tetrasulfonate | | | 1 | | | |
| Butyl Methoxydibenzoylmethane | 3,5 | 0,9 | 4 | 4,7 | | |
| Ethylhexyl Methoxycinnamate | 7,5 | 0 | 7,5 | 0 | 7,5 | 0 |
| Ethylhexylglycerin | 0,5 | 0,5 | 0,5 | 0,5 | 0,5 | 0,5 |
| Butylene Glycol | 5 | 5 | 5 | 5 | 5 | 5 |
| Glycerin | 7,5 | 5 | 7,5 | 5 | 7,5 | 5 |
| Isopropyl Stearate | 3 | 6 | 3 | 6 | 3 | 6 |
| Butylene Glycol Dicaprylate/Dicaprate | 7,5 | 8 | 7,5 | 8 | 7,5 | 8 |
| C12-15 Alkyl Benzoate | 9,5 | 5 | 9,5 | 5 | 9,5 | 5 |
| Parfum | q.s. | q.s. | q.s. | q.s. | q.s. | q.s. |
| Sodium Starch Octenylsuccinate | 0,4 | 0,55 | 0,4 | 0,55 | 0,4 | 0,55 |
| Titanium Dioxide | 4,5 | 2,5 | 5 | 6 | 5 | 6 |
| Magnesium Sulfate | 0,3 | 0,2 | 0,3 | 0,2 | 0,3 | 0,2 |
| Cetyl Dimethicone | 0 | 0,5 | 0 | 0,5 | 0 | 0,5 |
| Glycine | 0,2 | 0,5 | 0,2 | 0,5 | 0,2 | 0,5 |
| C18-36 Acid Triglyceride | 0,5 | 1 | 0,5 | 1 | 0,5 | 1 |
| Aqua | ad 100 | ad 100 | ad 100 | ad 100 | ad 100 | ad 100 |
| Ligninsulfonate | 1 | 2,5 | 1,5 | 4 | 3 | 3,5 |
| Creatine | | 0,5 | | | | |
| Creatinine | | 0,05 | | | | |
| Taurine | 1 | | | | | |
| Tocopheryl Acetate | 0,5 | 0,5 | 0,5 | 0,5 | 0,5 | 0,5 |
| Panthenol | 0 | 0,75 | 0 | 0,75 | 0 | 0,75 |

### W/O-Emulsionen: sehr hoher oder ultra hoher Schutz (very high or ultra high protection), SPF 30/40/50/50+

| **INCI** | **1** | **2** | **3** | **4** | **5** | **6** | **7** | **8** |
|---|---|---|---|---|---|---|---|---|
| Alcohol Denat. | 1 | 2 | 3 | 0 | 3 | 2 | 3 | 2 |
| Polyglyceryl-2 Dipolyhydroxystearate | 4 | 5,8 | 5 | 0 | 5 | 0 | 5 | 0 |
| PEG-30 Dipolyhydroxystearate | 0 | 0 | 0 | 3,5 | 0 | 3,5 | 0 | 3,5 |
| Trisodium EDTA | 1 | 1 | 1 | 1 | 1 | 1 | 1 | 1 |
| Preservative system | q.s. | q.s. | q.s. | q.s. | q.s. | q.s. | q.s. | q.s. |
| Diethylhexyl Butamido Triazone | 0,5 | | 1 | | 0 | 2 | 0 | 2 |
| Ethylhexyl Triazone | | 0,5 | | 2 | | | | |
| Bis-Ethylhexyloxyphenol Methoxyphenyl Triazine | 1 | 2 | 3 | 2,5 | 3 | 2,5 | 3 | 2,5 |
| 2,4-bis-[5-1(dimethylpropyl)benzoxazol-2-yl-(4-phenyl)-imino]-6-(2- ethylhexyl)-imino-1,3,5-triazin | | 1 | | | | | 5,9 | 9,4 |
| 2-(4'-Diethylamino-2'-hydoxybenzoyl)-benzoe-säurehexylester | | | 0,5 | | | | 9,5 | 9,3 |
| Octocrylene | 2,5 | 3,5 | 2,5 | 3 | 10 | 3 | 10 | 3 |
| Terephthalidene Dicamphor Sulfonic Acid | | | | | 9,5 | | 9,5 | |
| Drometrizole Trisiloxane | | | | | 12 | | 12 | |
| Disodium Phenyl Dibenzimidazole Tetrasulfonate | 0,5 | | | | | | | 1 |
| Butyl Methoxydibenzoylmethane | 3,5 | 4,5 | 0,9 | 4 | 4,7 | 4 | 2 | |
| Ethylhexyl Methoxycinnamate | 7,5 | 7,5 | 0,5 | 0,5 | 0,5 | 0,5 | 0,5 | 0,5 |
| Ethylhexylglycerin | 0,5 | 0,5 | 0,5 | 0,5 | 0,5 | 0,5 | 0,5 | 0,5 |
| Butylene Glycol | 2,5 | 2,5 | 5 | 4 | 5 | 4 | 5 | 4 |
| Glycerin | 7,5 | 7,5 | 5 | 6 | 5 | 6 | 5 | 6 |
| Isopropyl Stearate | 4 | 2,5 | 8 | 5 | 8 | 5 | 8 | 5 |
| Butylene Glycol Dicaprylate/Dicaprate | 7 | 6,5 | 8 | 6 | 8 | 6 | 8 | 6 |
| C12-15 Alkyl Benzoate | 9 | 7 | 8 | 5 | 8 | 5 | 8 | 5 |
| Parfum | q.s. | q.s. | q.s. | q.s. | q.s. | q.s. | q.s. | q.s. |
| Titanium Dioxide | 4,5 | 5 | 4,5 | 6 | 10 | 6 | 10 | 6 |
| Ligninsulfonat | 1,5 | 2,5 | 1,5 | 4,5 | 3 | 3,5 | 3,8 | 2,5 |
| Magnesium Sulfate | 0,3 | 0,1 | 0,2 | 0,2 | 0,2 | 0,2 | 0,2 | 0,2 |
| Cetyl Dimethicone | 0 | 0,5 | 0 | 0,2 | 0 | 0,2 | 0 | 0,2 |
| Dihydroxyacteon | | | 5 | | | | | |
| Glycine | 0,3 | 0,4 | 0,5 | 0,5 | 0,5 | 0,5 | 0,5 | 0,5 |
| C18-36 Acid Triglyceride | 0,4 | 0,6 | 0,5 | 0,4 5 | 0,5 | 0,4 5 | 0,5 | 0,4 5 |
| Tocopheryl Acetate | 0,5 | 0,5 | 0,5 | 0,5 | 0,5 | 0,5 | 0,5 | 0,5 |
| Panthenol | 0 | 0 | 1,5 | 1,5 | 1,5 | 1,5 | 1,5 | 1,5 |
| Aqua | ad 100 | ad 100 | ad 100 | ad 100 | ad 100 | ad 100 | ad 100 | ad 100 |

### Lichtschutzemulsionen: sehr hoher Schutz (very high protection) SPF 30/40/50

| **INCI** | **1** | **2** | **3** | **4** | **5** | **6** |
|---|---|---|---|---|---|---|
| Alcohol Denat. | 3 | 3 | 0 | 3 | 0 | 0 |
| Glyceryl Stearate SE | 1,5 | 1 | 0 | 1 | 0 | 0 |
| PEG-30 Dipolyhydroxystearate | 1 | 0 | 0 | 0 | 0 | 0 |
| Cetearyl Alcohol + PEG-40 Castor Oil + Sodium Cetearyl Sulfate | 3,75 | 2,5 | 0 | 2,5 | 0 | 0 |
| Cetyl PEG/PPG-10/1 Dimethicone | 0 | 0,8 | 3,8 | 0,8 | 3,8 | 3,8 |
| Polyglyceryl-2 Dipolyhydroxystearate | 0 | 0 | 2 | 0 | 2 | 2 |
| Trisodium EDTA | 1 | 1 | 0 | 1 | 0 | 0 |
| Preservative system | q.s. | q.s. | q.s. | q.s. | q.s. | q.s. |
| Cera Microcristallina + Paraffinum Liquidum | 0 | 0 | 3 | 0 | 3 | 3 |
| 2,4-bis-[5-1(dimethylpropyl)benzoxazol-2-yl-(4-phenyl)-imino]-6-(2- ethylhexyl)-imino-1,3,5-triazin | | | | 8,9 | 8,1 | 8,9 |
| 2-(4'-Diethylamino-2'-hydoxybenzoyl)-benzoe-säurehexylester | | | | 5,9 | 6,4 | 9 |
| Terephthalidene Dicamphor Sulfonic Acid | | | | | | 9,2 |
| Drometrizole Trisiloxane | | | | | | 13 |
| Glycerin | 7,5 | 3 | 5 | 3 | 5 | 5 |
| Butylene Glycol | 0 | 0 | 5 | 0 | 5 | 5 |
| Hydrogenated Coco-Glycerides | 0,5 | 0,5 | 0 | 0,5 | 0 | 0 |
| Dicaprylyl Carbonate | 1 | 2,35 | 0 | 2,35 | 0 | 0 |
| Caprylic/Capric Triglyceride | 5 | 4 | 7 | 4 | 7 | 7 |
| Butylene Glycol Dicaprylate/Dicaprate | 7,5 | 6,5 | 6 | 8 | 6 | 6 |
| C12-15 Alkyl Benzoate | 0 | 0 | 1,5 | 0 | 1,5 | 1,5 |
| Dicaprylyl Ether | 0 | 0 | 5 | 0 | 5 | 5 |
| Parfum | 0 | 0 | q.s. | 0 | q.s. | q.s. |
| Zinc Oxide + Dimethicone | 7 | 8 | 8 | 15 | 10 | 10 |
| Titanium Dioxide | 8 | 9 | 9 | 20 | 15 | 15 |
| Ligninsulfonat | 2, 5 | 4,5 | 1,5 | 1,5 | 3 | 3,5 |
| Magnesium Sulfate | 0 | 0 | 0,3 | 0 | 0,3 | 0,3 |
| Cyclomethicone | 0 | 0 | 4 | 0 | 4 | 4 |
| Glycine | 0 | 0 | 0,5 | 0 | 0,5 | 0,5 |
| Xanthan Gum | 0,2 | 0,3 | 0 | 0,3 | 0 | 0 |
| C18-36 Acid Triglyceride | 0,5 | 1 | 0 | 1 | 0 | 0 |
| Cetyl Alcohol | 1 | 1,5 | 0 | 1,5 | 0 | 0 |
| Aqua | ad 100 | ad 100 | ad 100 | ad 100 | ad 100 | ad 100 |
| Tocopheryl Acetate | 0,5 | 0,5 | 0,5 | 0,5 | 0,5 | 0,5 |
| Dioic acid | 0,1 | | | | | |
| Panthenol | 0 | 0 | 1,4 | 0 | 1,4 | 1,4 |

## Patentansprüche

1. Kosmetische Zubereitung enthaltend
a) ein oder mehrere UV-Lichtschutzfilter sowie
b) Ligninsulfonsäure und/oder Ligninsulfonate.

2. Kosmetische Zubereitung nach Anspruch 1, **dadurch gekennzeichnet, dass** die Zubereitung UV-Lichtschutzfilter in einer Konzentration von 0,1 bis 30 Gewichts-%, bezogen auf das Gesamtgewicht der Zubereitung, enthält.

3. Kosmetische Zubereitung nach einem der Ansprüche 1 bis 2, **dadurch gekennzeichnet, dass** die Zubereitung Ligninsulfonsäure und/oder Ligninsulfonate in einer Gesamtkonzentration von 0,01 bis 20 Gewichts-%, bezogen auf das Gesamtgewicht der Zubereitung, enthält.

4. Kosmetische Zubereitung nach einem der Ansprüche 1 bis 3, **dadurch gekennzeichnet, dass** als Ligninsulfonat Natriumligninsulfonat (CAS 8061-51-6) eingesetzt wird.

5. Kosmetische Zubereitung nach einem der Ansprüche 1 bis 4, **dadurch gekennzeichnet, dass** das Ligninsulfonat ein durchschnittliches Molekulargewicht von bis zu 10000 Dalton aufweist.

6. Kosmetische Zubereitung nach einem der Ansprüche 1 bis 5, **dadurch gekennzeichnet, dass** die UV-Lichtschutzfilter gewählt werden aus der Gruppe der UV-Lichtschutzfilter 2,4-Bis-{[4-(2-ethyl-hexyloxy)-2-hydroxy]-phenyl}-6-(4-methoxyphenyl)-1,3,5-triazin; Dioctylbutylamidotriazon; 2,4-bis-[5-1 (dimethylpropyl)benzoxazol-2-yl-(4-phenyl)-imino]-6-(2-ethylhexyl)-imino-1,3,5-triazin; 4,4',4"-(1,3,5-Triazin-2,4,6-triyitriimino)-tris-benzoesäure-tris(2-ethylhexylester); 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; Ethylhexylsalicylat; Terephthalidendicamphersulfonsäure; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalon-säuredi(2-ethylhexyl)-ester; Dioctylbutylamidotriazon; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dimethicodiethylbenzalmalonat; 4-Methoxyzimtsäure(2-ethylhexyl)ester; 4-(tert.-Butyl)-4'-methoxydibenzoylmethan; Titandioxid; Zinkoxid enthält.

7. Kosmetische Zubereitung nach einem der Ansprüche 1 bis 6, **dadurch gekennzeichnet, dass** ein oder mehrere UV-Lichtschutzfilter gewählt werden aus der Gruppe der Triazine, Titandioxid.

8. Kosmetische Zubereitung nach einem der Ansprüche 1 bis 7, **dadurch gekennzeichnet, dass** die Zubereitung den UV-Lichtschutzfilter 4-(tert.-Butyl)-4'-methoxydibenzoylmethan (CAS-Nr. 70356-09-1) enthält.

9. Kosmetische Zubereitung nach einem der vorhergehenden Ansprüche, **dadurch gekennzeichnet, dass** die Zubereitung Selbstbräuner enthält.

10. Kosmetische Zubereitung nach einem der vorhergehenden Ansprüche, **dadurch gekennzeichnet, dass** die Zubereitung in Form einer Emulsion vorliegt.
